# EUROPEAN PATENT APPLICATION

(11) **EP 2 799 877 A1**
(43) Date of publication of application: **05.11.2014**
(21) Application number: 13166349.4
(22) Date of filing: 03.05.2013
(51) Int. Cl.: G01N 33/68

(54) **Process for diagnosing a human subject with diseases affecting the kidneys, or at risk of acquiring diseases affecting the kidneys**

(71) Applicant: Universität Ulm, 89081 Ulm (DE)
(72) Inventor: Muench, Jan, 89233 Neu-Ulm (DE); Kirchhoff, Frank, 89077 Ulm-Einsingen (DE); Zirafi, Onofrio, 89555 Steinheim (DE); Mohr, Katharina, 89077 Ulm (DE)
(74) Representative: Keller, Günter

(57) **Abstract**

The present invention relates to an *in vitro* process for the immunological diagnosis of a human subject with regard to diseases affecting the kidneys or a risk of acquiring diseases affecting the kidneys or graft-versus-host disease or at risk of acquiring graft-versus-host disease by determining immunologically the presence and/or concentration of the peptide ALB408-423 (SEQ ID NO 1) in a sample comprising the steps of
(a) contacting the sample with a capture reagent which is capable of specifically binding to ALB408-423 (SEQ ID NO 1),
(b) determining the presence and/or amount of ALB408-423 (SEQ ID NO 1) bound to capture reagent formed in step (a).

SEQUENCE LISTING

<110>  Universitätsklinikum Ulm
       Münch, Jan
       Zirafi, Onofrio
       Borczuch, Katharina
       Kirchhoff, Frank

<120>  Process for diagnosing a human subject with diseases affecting
       the kidneys, or at risk of acquiring diseases affecting the
       kidneys

<130>  PEP02321UN1

<160>  3

<170>  PatentIn version 3.5

<210>  1
<211>  16
<212>  PRT
<213>  Artificial Sequence

<220>
<223>  ALB408-423

<400>  1

Leu Val Arg Tyr Thr Lys Lys Val Pro Gln Val Ser Thr Pro Thr Leu
1               5                   10                  15


<210>  2
<211>  8
<212>  PRT
<213>  Artificial Sequence

<220>
<223>  peptide used for generation of capture reagent

<400>  2

Leu Val Arg Tyr Thr Lys Lys Cys
1               5


<210>  3
<211>  9
<212>  PRT
<213>  Artificial Sequence

<220>
<223>  peptide used for generation of the first detecting reagent

<400>  3

Cys Pro Gln Val Ser Thr Pro Thr Leu
1               5

## Description

### Field of the invention

The present invention relates to a process for diagnosing a human subject with diseases affecting the kidneys or at risk of acquiring diseases affecting the kidneys or graft-versus-host disease or at risk of acquiring graft-versus-host disease by determining the concentration of a peptide having the designation of ALB408-423 (SEQ ID NO 1) in a sample such as urine, blood, plasma, serum, vaginal lavage, and or cerebrospinal fluid.

ALB408-423 is a 16 amino acid peptide with the following sequence: LVRYTKKVPQVSTPTL, and a molecular weight of about 1,830 Da (SEQ ID NO 1). This peptide corresponds exactly to residues 408 to 423 of mature human serum albumin (hSA) and has been found in patients with diseases affecting the kidneys, graft-versus-host disease, and in serum of healthy individuals. Thus, ALB408-423 (SEQ ID NO 1) can be used as a diagnostic marker to screen patients with diseases affecting the kidneys or at risk of acquiring diseases affecting the kidneys. In the same manner ALB408-423 (SEQ ID NO 1) can be used as a diagnostic marker for patients with graft-versus-host disease, or at risk of acquiring graft-versus-host disease.

### Background of the invention

Chalmers et al. Anal. Chem. 2005, 77, 7163-7171, analyzed urine of patients with focal-segmental glomerulosclerosis, membranous glomerulonephritis, minimal change disease, IgA nephropathy and diabetic nephropathy for signature polypeptides with the use of capillary electrophoresis and mass spectrometry. One of the albumin fragments identified as marker for renal disease was a peptide with the following sequence: LVRYTKKVPQVSTPTL. The authors suggest that the appearance of albumin fragments in urine might serve a more specific and earlier marker of renal diseases and injuries than the presence of the parental-albumin protein itself. It is generally accepted that the albumin present in the lumen of the proximal tubule is largely reabsorbed at the apical side of proximal tubular cells by a receptor complex consisting of megalin and cubulin, then degraded into polypeptides and amino acids by the tubular cells, and reutilized by the body. Small quantities of albumin escape this metabolism and appear intact even in the urine of healthy individuals. With progressive kidney disease, the quantity of albumin passing the injured glomerular filter increases and finally saturates the reabsorption capacity of the proximal tubule, as reflected by a pathological albumin excretion rate. However, albumin in large amounts has been shown to alter activity of intracellular enzymes including proteases. Thus, it is proposed that the appearance of small albumin fragments and peptides is caused by initial changes in proteolytic activity associated with the onset of renal disease, and the identified albumin fragments may serve as potential early markers for renal damage indicating the inability of proximal renal tubular cells to cope with the increased tubular albumin flux.

Theodorescu et al. Electrophoresis, 2005, 26, 2797-2808 used capillary electrophoresis coupled with mass spectrometry to analyze urine of patients affected with IgA nephropathy, diabetic nephropathy, focal segmental glomerulosclerosis, minimal change disease, membranous glomerulonephritis, renal transplant and renal cancer, looking specifically for peptide signatures that could be used as a diagnostic tool for such diseases. Here too, the same albumin fragment (LVRYTKKVPQVSTPTL) was identified.

Kausler and Spiteller Biol. Chem. Hoppe Seyler, 1991, 372, 849-855, using various chromatographic means and mass spectrometry, analyzed hemofiltrate of uremic patients and also reported presence of the above mentioned albumin fragment (LVRYTKKVPQVSTPTL).

Kaiser et al., Blood, 2004, 104(2), 340-349, used capillary electrophoresis and mass spectrometry to analyze urine of hematopoietic stem cell transplantation patients. Here again, the peptide with the sequence LVRYTKKVPQVSTPTL was detected and identified as the marker for early detection of graft-versus-host disease before the onset of the clinical symptoms.

Aristoteli et al, Journal of Proteome Research, 2007, 6, 571-581 used mass spectrometry to identify peptides in plasma of healthy individuals. Once more, the above mentioned albumin fragment (LVRYTKKVPQVSTPTL) was detected.

DE 102007030904 and WO2009004054 disclose the isolation, characterization and synthesis of ALB408-423 (LVRYTKKVPQVSTPTL) and its role as a CXCR4 antagonist.

The prior art, discloses cumbersome and time consuming methods, like capillary electrophoresis, different mass spectrometry techniques, and various chromatographic methods to identify the albumin fragment ALB408-423 (SEQ ID NO 1) in a sample. These methods are not suitable for large volume screening of patients, and do not allow for fast and easy identification and quantification of such marker peptides.

Diseases affecting the kidneys represent a growing health problem among the general population. It is believed, that this trend is caused by the rise of diagnosed diabetes, increase in general life expectancy, and survival from other diseases. Current diagnostic tools like urinalysis and blood test are mainly indirect and kidney biopsy is often required. Consequently, the disease is frequently diagnosed in later stages, and thus the success rate of existing therapies is decreased.

Recently, low molecular weight peptides arising from fragmentation of abundant blood proteins gained more interest for the diagnostic utility. With the use of proper technology diagnosis of a variety of diseases affecting the kidneys can be based on indicative changes in the presence of polypeptide markers.

Another difficulty arises from the source of the samples, like blood, serum, plasma, cerebral fluid, vaginal lavage and urine which can make it difficult to accurately identify and quantify low weight peptides. Such peptides can be masked or have a very short half-life, thus there is a need for a quick, accurate and efficient method to identify and quantify the presence of the albumin fragment ALB408-423 (SEQ ID NO 1) in a sample.

It is therefore an object of the present invention to provide methods for semi- quantitative or quantitative measurement of ALB408-423 (SEQ ID NO 1) concentration in a sample such as urine, blood, plasma, serum, vaginal lavage, and or cerebral fluid. The results of the process can be used for diagnosing a human subject with diseases affecting the kidneys or at risk of acquiring diseases affecting the kidneys or graft-versus-host disease or at risk of acquiring graft-versus-host disease.

### Summary of the invention

The present invention relates to an *in vitro* process for the immunological diagnosis of a human subject with regard to diseases affecting the kidneys or a risk of acquiring diseases affecting the kidneys by determining immunologically the presence and/or the concentration of the peptide ALB408-423 (SEQ ID NO 1) in a sample comprising the steps of:
(a) contacting the sample with a capture reagent which is capable of specifically binding to ALB408-423 (SEQ ID NO 1),
(b) determining the presence and/or amount of ALB408-423 (SEQ ID NO 1) bound to capture reagent formed in step (a).

In one embodiment, the present invention provides a process for diagnosing a human subject with a disease affecting the kidneys selected from the group comprising: any renal disease, nephropathy, IgA nephropathy, proteinuria, uremia and membranous glomerulonephritis.

In another embodiment, the present invention provides a process for diagnosing a human subject with a graft-versus-host disease or a risk of acquiring graft-versus-host disease.

In a preferred embodiment, the method of the invention comprises only steps which are carried out *in vitro.* In that embodiment, the step of obtaining the sample from the human subject is not encompassed by the present invention.

The term "sample" as used herein designates a composition which is derived from the body of a human subject. Preferred samples are urine, blood, plasma, serum, vaginal lavage, semen, sweat, milk and/or cerebral fluid. The sample may be a composition which has been processed to be in a condition suitable for diagnosis with the method according to the invention. For example, a blood sample may be subject to centrifugation such that serum is obtained, or a urine sample may be diluted prior to analysis in a preferred dilution range of 10 to 10,000 fold. In especially preferred embodiments the dilution range is from 10 to 100 fold. The process may include centrifugation, precipitation, concentration, filtration, dialysis, and or dilution. The type of processing may depend on the technique which is used for detecting the ALB408-423 (SEQ ID NO 1) in a sample and from the nature of the sample.

The process of present invention may be performed as a direct or indirect immunoassay selected from the group comprising: competitive binding assay, non-competitive binding assay, a radioimmunoassay, an enzyme-linked immunosorbent assay (ELISA), a sandwich assay, a gel diffusion immunodiffusion assay, an agglutination assay, a fluorescent immunoassay, a chemiluminescence immunoassay, a protein A or protein G immunoassay, an immunoelectrophoresis assay, Western Blot, Dot Blot and flow cytometry.

In a preferred embodiment the present invention is a sandwich ELISA. Sandwich ELISAs are well known in the art; and a plate-based assay that uses antibodies to detect, identify and/or quantify substances such as peptides, proteins, antibodies and hormones is preferred.

Such sandwich ELISA can preferably be used to determine the concentration of ALB408-423 (SEQ ID NO 1) and to diagnose a human subject with graft-versus-host disease or risk of acquiring graft-versus-host disease.

As used herein, the term "antibody" designates an immunoglobin or a derivative thereof having the same binding specificity. The antibody according to the present invention may be a monoclonal antibody or an antibody derived from a polyclonal antiserum, whereby monoclonal antibodies are preferred. The antibody or the derivative thereof may be of natural origin or may be semi-synthetically produced. Such synthetic products also comprise non-proteinaceous material that has the same or essentially the same binding specificity as the antibody of the invention. Such products may, for example be obtained by peptidomimetics. For the person skilled in the art it is well-known to produce monoclonal antibodies by using the hybridoma technology which was first described by Köhler and Milstein. In recent years much progress has been made and it is standard knowledge to design monoclonal antibodies which bind specifically to certain epitopes of a peptide.

It is not required that whole antibodies are used also binding fragments of antibodies like Fab fragments or single chain antigen binding fragments can be used. It is essential that the binding part of the antibody or antibody derivative binds specifically to the desired epitope.

The antibody of the present invention against ALB408-423 (SEQ ID NO 1) can also be produced by immunizing an animal such as rabbit, guinea pig, rat, goat, sheep or mouse with ALB408-423 or a fragment thereof or a derivative thereof by a method well known by those skilled in the art. The antigen to be used for immunization is ALB408-423 (SEQ ID NO 1) or a fragment thereof or a derivative thereof. The antigen may be prepared by recombinant expression in a host, e.g. in bacteria, insect cells, or mammalian cells. It may also be prepared by chemical synthesis such as the solid phase method. Techniques for producing monoclonal antibodies are known to those skilled in the art.

The antibody to be used in accordance with the present invention should be specific to ALB408-423 (SEQ ID NO 1), i.e. it does not recognize other peptides or proteins in the sample. This is an important feature since undesired cross-reactions cause false results.

In the process of present invention the capture reagent is a monoclonal or polyclonal antibody, preferably a monoclonal antibody, which specifically binds to an epitope present in ALB408-423 (SEQ ID NO 1). The capture reagent is preferably immobilized on a carrier. Preferred carrier is a solid carrier, such as an ELISA plate container molded from a carrier polymer such as styrene or polyester.

In a preferred embodiment the capture antibody is immobilized on an ELISA titer plate. Alternatively, however, the capture reagent (antibody) can be bound to plastic beads. By using capture reagents bound to small plastic and/or magnetic beads it is possible to disperse the capture antibody attached to the beads into the sample and to easily separate the first intermediate complex formed by the capture antibody and the peptide to be detected. This intermediate complex can be easily separated from the sample and further processed like for example washed in order to remove unspecific binding.

In a preferred aspect of the present invention, step (b) further comprises the following steps:
(i) contacting the first intermediate complex obtained in step (a) with a first detecting reagent, which is capable of specifically binding to ALB408-423 (SEQ ID NO 1), resulting in a second intermediate complex whereby the first detecting reagent can produce a signal,
(ii) measuring the detectable signal produced in the second intermediate complex obtained in step (i), wherein the detectable signal corresponds to the concentration of ALB408-423 (SEQ ID NO 1) in a sample.

In one embodiment of the present invention the sample is contacted with the capture reagent to produce the first intermediate complex, wherein capture reagent is immobilized on a carrier, wherein the first intermediate complex is the sample contacted with the capture reagent. If the sample contains ALB408-423 (SEQ ID NO 1), the intermediate complex includes ALB408-423 bound to the capture reagent; if on the other hand, the sample does not contain ALB408-423 the intermediate complex does not include ALB408-423 bound to the capture reagent. The first intermediate complex may or may not include ALB408-423 bound to the capture reagent. The first intermediate complex simply refers to the contacted sample to the capture reagent.

Another embodiment of the present invention comprises the contacting of the first intermediate complex with a first detecting reagent to produce the second intermediate complex, wherein the second intermediate complex is the sample contacted with the capture reagent and further contacted with the first detecting reagent. If the sample contains ALB408-423 (SEQ ID NO 1), the second intermediate complex includes bound ALB408-423 to the capture reagent and the first detecting reagent, forming a so-called sandwich structure. If, on the other hand, ALB408-423 is not present in the sample, the sandwich structure of the second intermediate complex cannot be formed. The formed sandwich structure can then be detected using e.g. color reactions and/or other detection reactions.

In another embodiment of the present invention, the first detecting reagent is a monoclonal or polyclonal antibody, preferably a monoclonal antibody, that specifically binds to one of the epitopes present on ALB408-423 (SEQ ID NO 1). The first detecting reagent may be labeled.

The first detecting reagent may be directly labeled by having for example a dye or a chemoluminescent structure. Alternatively the first detecting reagent may be coupled to a further detection reagent by using coupling means such as protein A or biotin.

In another embodiment the present invention involves contacting the second intermediate complex with the second detecting reagent to produce the third intermediate complex, wherein the third intermediate complex is the second intermediate complex contacted with the second detecting reagent. If the sample contains ALB408-423 (SEQ ID NO 1), the second intermediate complex includes bound ALB408-423 to the capture reagent and the first detecting reagent, forming a so called sandwich structure, wherein the second detecting reagent is bound to the first detecting reagent. If, on the other hand, ALB408-423 is not present in the sample, the sandwich structure of the third intermediate complex does not form.

In another embodiment of the present invention, the second detecting reagent is an anti-antibody that specifically binds to the first detecting reagent or a protein that is capable of binding to the first detecting reagent. The second detecting reagent may be labeled and detectable using processes of the present invention. The second detecting reagent used in the present invention can be selected from the group comprising: first-detecting-reagent-specific anti-antibody and/or biotin-specific protein (e.g. Streptavidin-HRP or Avidin-HRP).

The presence of the sandwich formed in the second intermediate complex, or the sandwich in the third intermediate complex can be detectable. The detectable signal that is produced by the second intermediate complex or the third intermediate complex can be selected from the group comprising: optical density, fluorescence, luminescence and/or radioactive signal. It is preferred that the detectible signal is optical density.

The step of detecting ALB408-423 (SEQ ID NO 1) in a sample may include determining the presence or absence of ALB408-423 in a qualitative manner. The step of detecting ALB408-423, however, may also include determining the amount or concentration of ALB408-423 in a sample in a quantitative or semi-quantitative manner. For a quantitative determination usually a calibration curve is required.

The capture reagent and the first detecting reagent of the present invention are preferably monoclonal antibodies, wherein capture reagent and the first detecting reagent each independently and specifically bind to different epitopes present in ALB408-423 (SEQ ID NO 1).

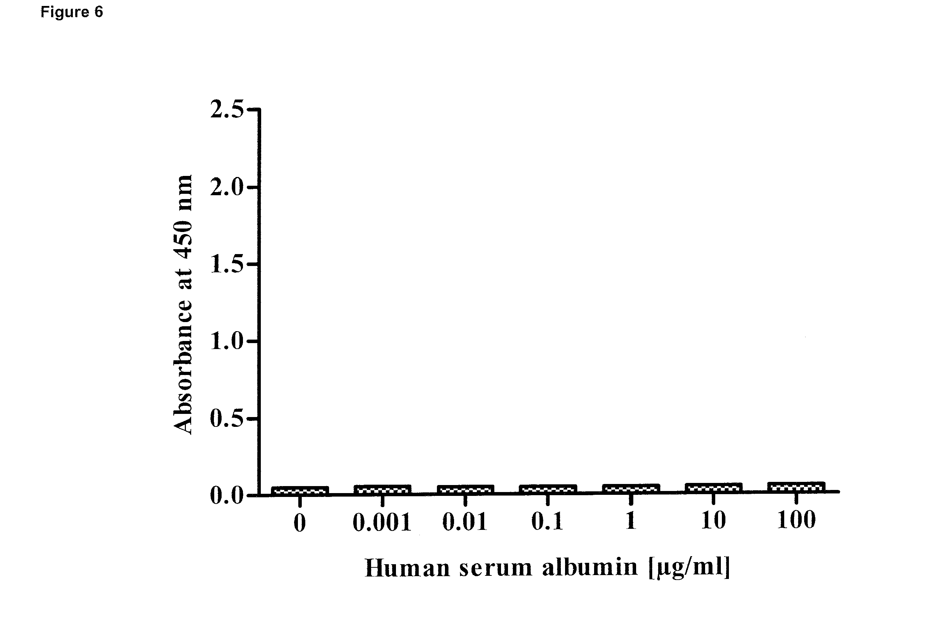

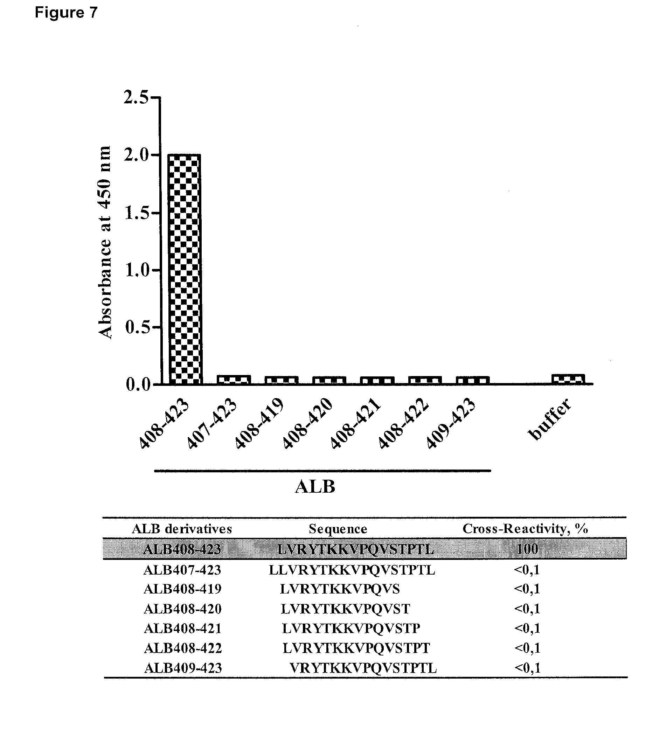

The major challenge in the detection of ALB408-423 (SEQ ID NO 1) in samples is that longer-length variants of the peptide are also present in samples such as plasma, making the specific detection of ALB408-423 difficult. Cross-reactions of indirect ELISAs with longer peptides, other analogous fragments, and/or hSA can complicate the detection of ALB408-423 (SEQ ID NO 1). The process of present invention proposes a highly sensitive and specific sandwich ELISA to detect ALB408-423 (SEQ ID NO 1) directly from the samples to diagnose a human subject with diseases affecting the kidneys or the risk of acquiring diseases affecting the kidneys. Figures 6 and 7 further illustrate the specificity of the ALB408-423 (SEQ ID NO 1) sandwich ELISA.

Also shorter-length variants of the ALB408-423 (SEQ ID NO 1) should not be immunologically detected with the process of the present invention (e.g. Figure 7). Such shorter-length variants are derivatives of the peptide ALB408-423 (SEQ ID NO 1) which lack from either the C- and/or N-terminus up to 7 amino acids. Such shorter-length variants have usually 8 up to 15 amino acids.

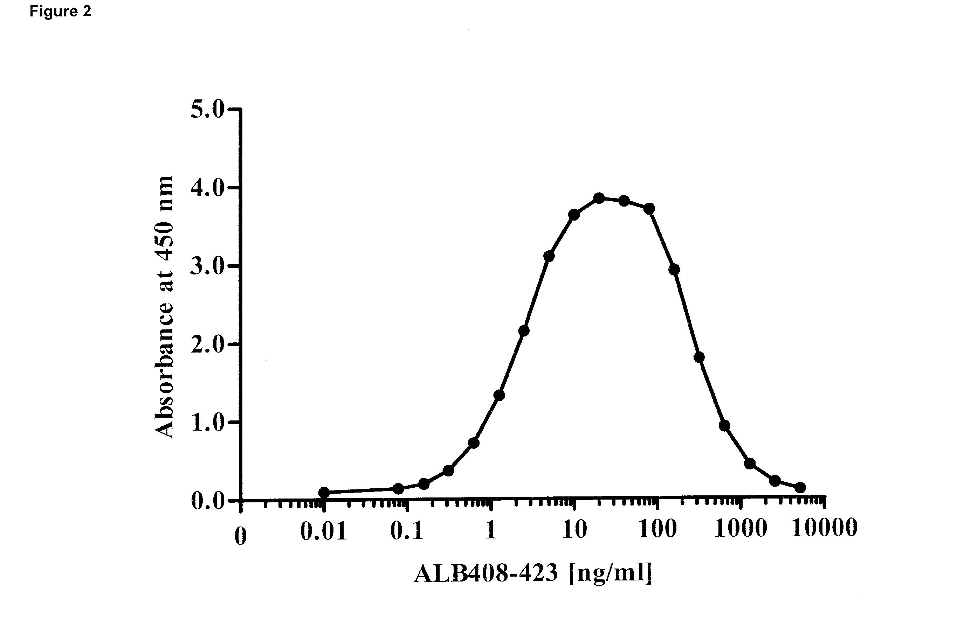

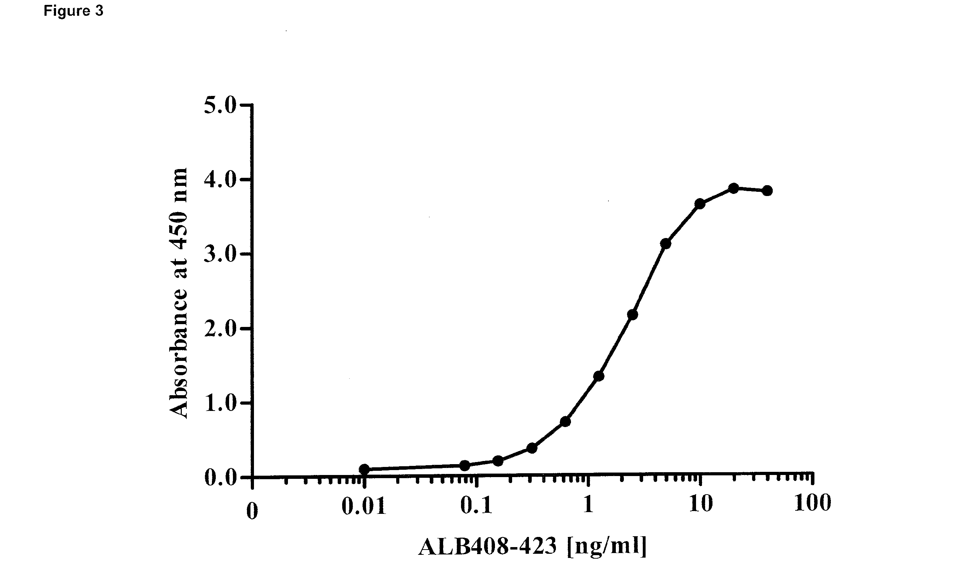

The sensitivity of the sandwich ELISA was analyzed using synthetic ALB408-423 (SEQ ID NO 1). The results show a peptide concentration-dependent increase of the ODs which reached a plateau (i.e. upper limit of detection) at 40-100 ng/ml whereas at higher peptide concentrations, the ODs decreased (Figure 2 and 3). This phenomenon is known as high dose hook or "hook effect" and is caused by an overwhelming amount of antigen (i.e. SEQ ID NO 1) that affects the binding capacity of the corresponding antibodies. The observed hook effect restricts the use of the ELISA as it might result in false negative results when peptide concentrations are higher than the upper detection limit. For this, it is important to dilute the sample from 10-fold to 100-fold, more preferably 10-fold. The detection limit of the present invention allows for a highly sensitive detection of the peptide ALB408-423 (SEQ ID NO 1).

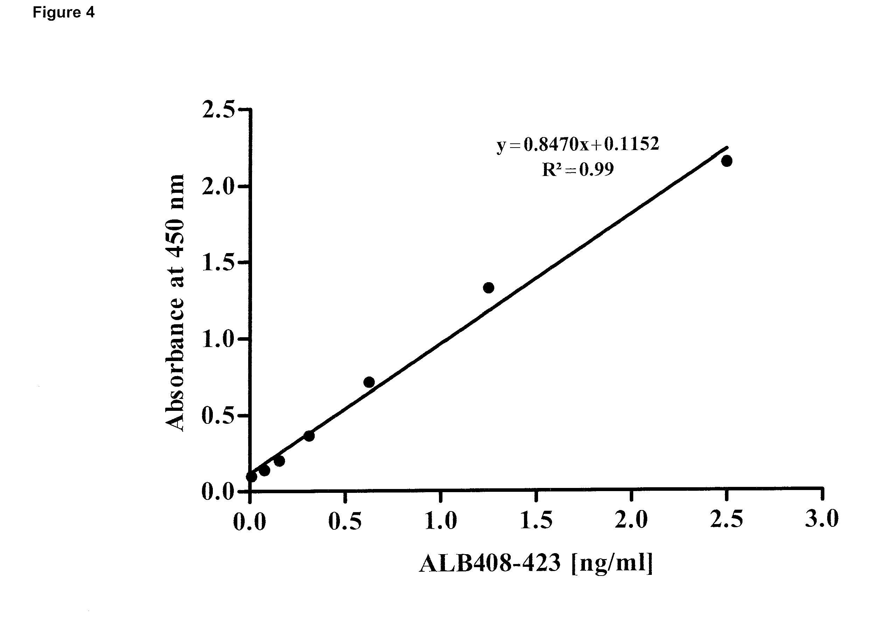

The detection range of ALB408-423 (SEQ ID NO 1) in a sample using the process of the present invention was determined by methods known in the art (Figure 4). In one embodiment of the present invention, the detection range for ALB408-423 (SEQ ID NO 1) is within 0.1 to 10 ng/ml, preferably 0.4 to 5 ng/ml, based on total volume of a sample.

Another aspect of the present invention is an immunoassay kit for diagnosing a human subject with regard to diseases affecting the kidneys or at risk of acquiring diseases affecting the kidneys by determining concentration of peptide ALB408-423 (SEQ ID NO 1). In the same manner, the immunoassay kit of the present invention can also be used for diagnosing a human subject with graft-versus-host disease or at risk of acquiring graft-versus-host disease. Preferably, the immunoassay kit is a sandwich ELISA test kit. It is preferred that the immunoassay kit of the present invention includes a capture reagent. In one embodiment, the capture reagent does not cross-react with other proteins present in the sample. The preferred antibodies of this kit have been described supra in connection with the process of the invention. The capture reagent may be immobilized on a solid phase such as a microtiter plate or a test strip. The kit may comprise a further antibody (i.e. first detecting reagent) capable of specifically binding to ALB408-423 (SEQ ID NO 1). The first detecting reagent (a monoclonal or a polyclonal antibody) may be labeled and may be used in a sandwich ELISA as known in the art. Enzyme labels, fluorescent labels, light emission labels, radioactive labels, etc. can be used. It is preferred that an enzyme label such as alkaline phosphatase, β-galactosidase or horse radish peroxidase is used in the process of the present invention.

The immunoassay kit of the present invention may further comprise other components such as a wash buffer concentrate, a composition comprising ALB408-423 (SEQ ID NO 1) as a standard, stop solution (e.g. HCl solution) and the like.

### Detailed description of the invention

The present invention is described in more detail in the figures and examples, whereby preferred embodiments of the ALB408-423-specific sandwich ELISA are disclosed.

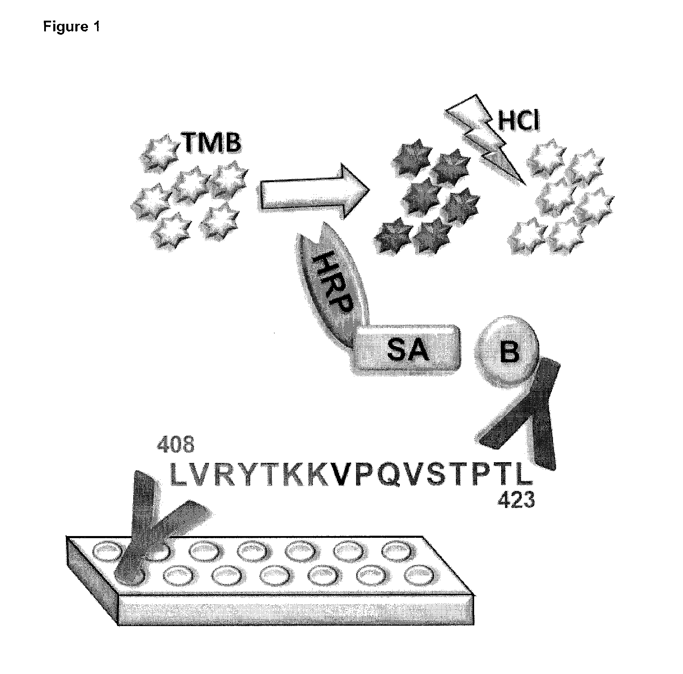

Figure 1: Shows a diagram that demonstrates the principle of the ALB408-423 (SEQ ID NO 1) sandwich ELISA of the present invention.
Figure 2: Establishment of the ALB408-423 (SEQ ID NO 1) sandwich ELISA. 2-fold dilutions of 5 µg/ml ALB408-423 were prepared in assay buffer. The dilutions were measured via the ALB408-423 (SEQ ID NO 1) sandwich ELISA at 450 nm vs. 650 nm reference wavelength. The shown result is representative.
Figure 3: Establishment of the ALB408-423 (SEQ ID NO 1) sandwich ELISA. 2-fold dilutions of 40 ng/ml ALB408-423 were prepared in assay buffer. The dilutions were measured via the ALB408-423 sandwich ELISA at 450 nm vs. 650 nm reference wavelength.
Figure 4: Calibration curve of an ALB408-423 (SEQ ID NO 1) sandwich ELISA. 2-fold dilutions of 5 ng/ml ALB408-423 down to 0.078 ng/nl were prepared in assay buffer and analyzed by ALB408-423 (SEQ ID NO 1) sandwich ELISA at 450 nm vs. 650 nm reference wavelength. In the absence of spiking, no signal was detected. The linear equation (y = m*x + b) with constant (slope, m and y-intercept, b) and variable values (independent x-value, dependent y-value) aids calculation of unknown sample concentration. The R² value is the coefficient of determination providing information on the variability of the values. R² values (0-1) close to 1 indicate low variability of the analysis (n=2, results shown are representative).

The linear range of the detection method of the present invention lies between 0.4 - 0.5 ng/ml. The standard concentrations used in the examples of the present invention all fall within this range.

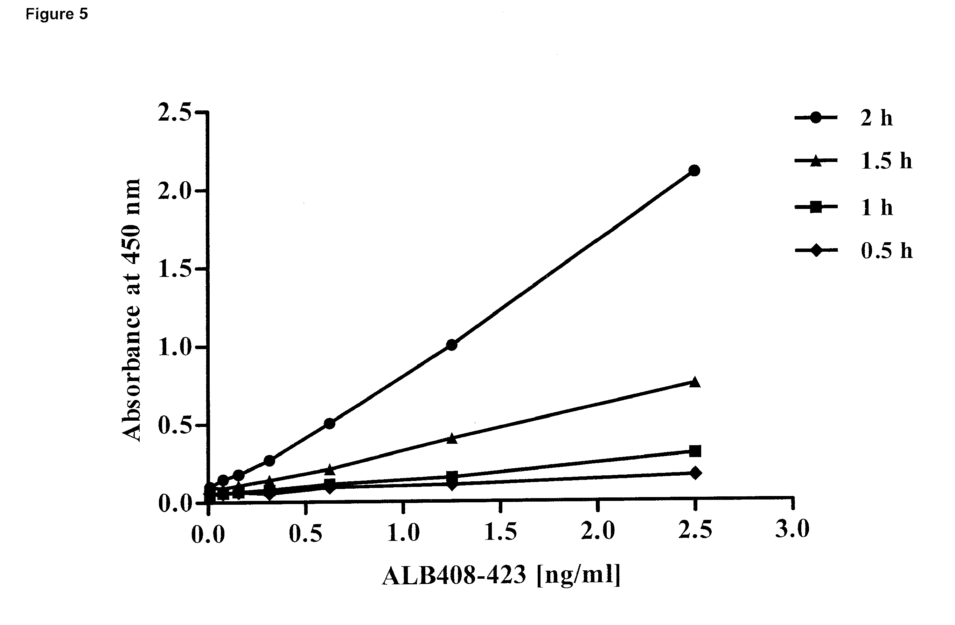

Figure 5: Determining the amount of time needed for the incubation of samples with the first detecting reagent. Two hours of incubation of samples with the first detecting reagent is necessary to achieve relevant signal strength in the ALB408-423 (SEQ ID NO 1) sandwich ELISA. 2-fold dilutions of 2.5 ng/ml ALB408-423 down to 0.078 ng/ml were prepared in assay buffer and analyzed by ALB408-423 (SEQ ID NO 1) sandwich ELISA at 450 nm vs. 650 nm reference wavelength.
Figure 6: The ALB408-423 (SEQ ID NO 1) sandwich ELISA does not cross react with human serum albumin. 10-fold dilutions of 100 µg/ml human serum albumin down to 0.001 µg/ml were prepared in assay buffer and analyzed by ALB408-423 (SEQ ID NO 1) sandwich ELISA at 450 nm vs. 650 nm reference wavelength.
Figure 7: The ALB408-423 (SEQ ID NO 1) sandwich ELISA detects ALB408-423 (SEQ ID NO 1) only, and no shorter or longer variants thereof. 2.5 ng/ml of different ALB length variants were prepared in assay buffer. The samples were analyzed by ALB408-423 (SEQ ID NO 1) sandwich ELISA at 450 nm vs. 650 nm reference wavelength. In the absence of spiking, no signal was detected.

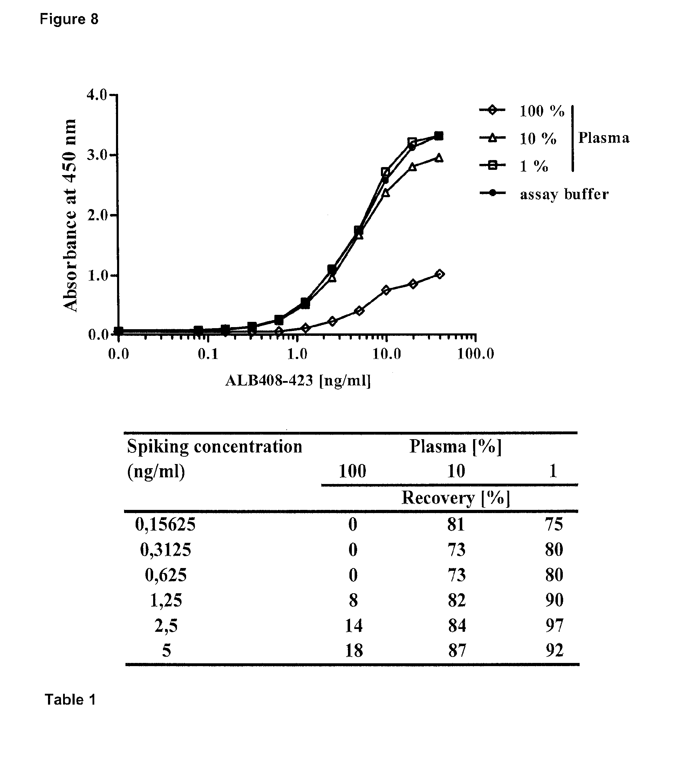

Figure 8 and Table 1: Comparison of the standard curve prepared in assay buffer and in human plasma. 2-fold dilutions of 5 ng/ml ALB408-423 (SEQ ID NO 1) down to 0.078 ng/ml were prepared in 0, 1, 10 and 100% human plasma. The samples were analyzed by ALB408-423 (SEQ ID NO 1) sandwich ELISA at 450 nm vs. 650 nm reference wavelength.

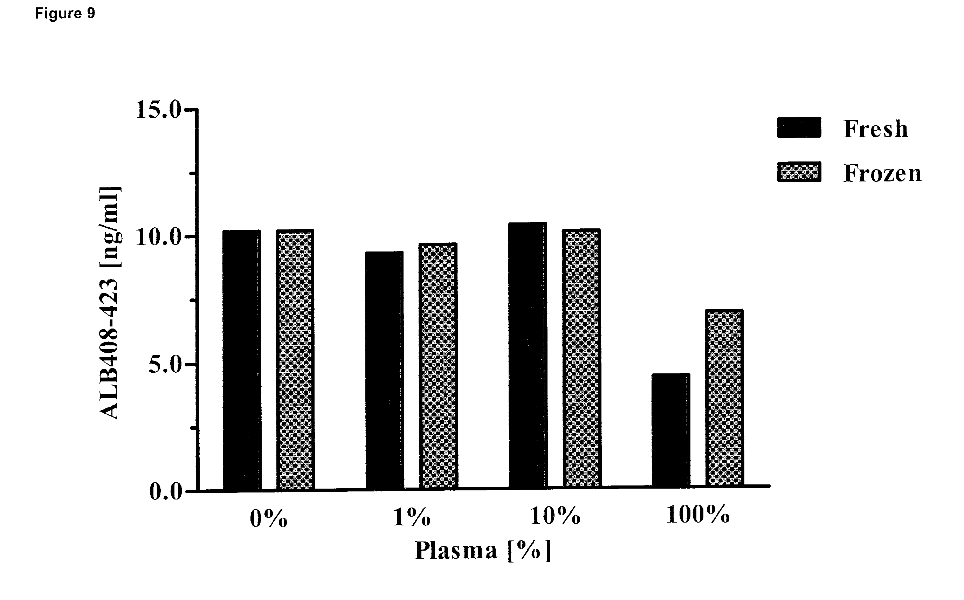

Figure 9: Recovery of ALB408-423 (SEQ ID NO 1) (10 ng/ml) in fresh and frozen human plasma (1-100%) compared to ALB408-423 (SEQ ID NO 1) in assay buffer. 2-fold dilutions of 5 ng/ml ALB408-423 (SEQ ID NO 1) down to 0.078 ng/ml were spiked in 0, 1, 10 and 100% human plasma. The samples were analyzed by ALB408-423 (SEQ ID NO 1) sandwich ELISA at 450 nm vs. 650 nm reference wavelength.

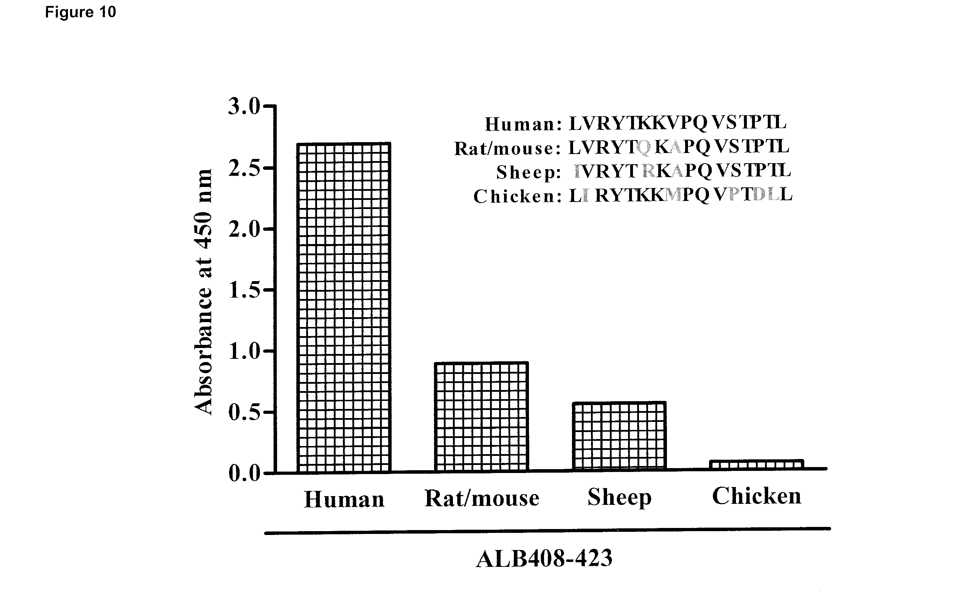

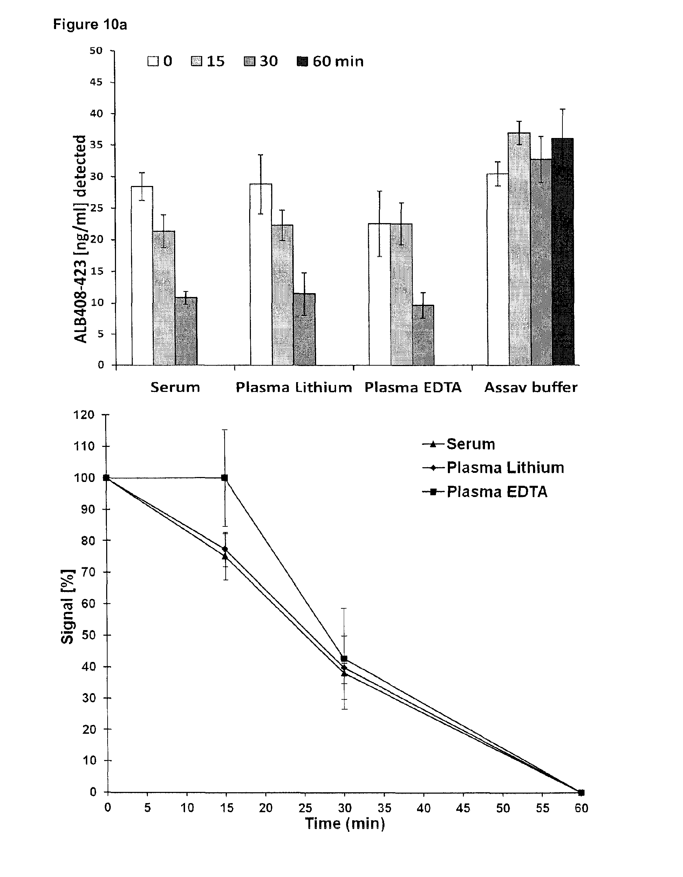

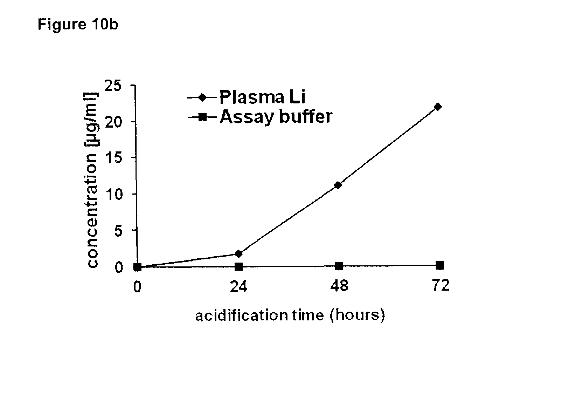

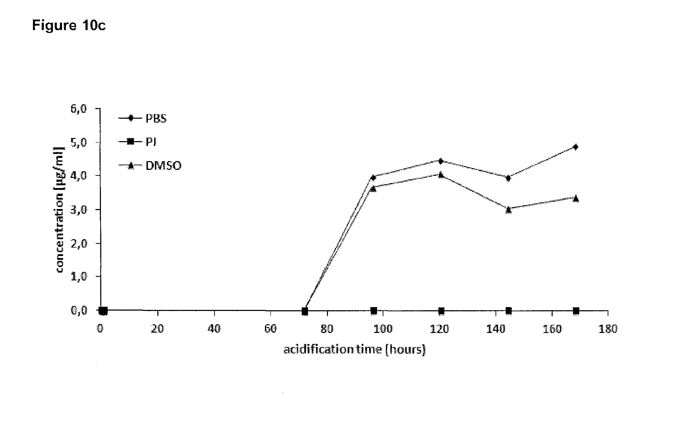

Figure 10: The ALB408-423 (SEQ ID NO 1) sandwich ELISA detects human ALB408-423 (SEQ ID NO 1) more specifically, compared to ALB408-423 from other species. 5 ng/ml of ALB408-423 from different species were prepared in assay buffer. The samples were analyzed by ALB408-423 (SEQ ID NO 1) sandwich ELISA at 450 nm vs. 650 nm reference wavelength. Sequence differences are indicated in grey color.
Figure 10a: Determination of the "Half-Life" of synthetic ALB408-423 (SEQ ID NO 1). Fresh plasma and serum were spiked with 50 ng/ml ALB408-423 (SEQ ID NO 1), then incubated at 37°C and 5% CO₂ for various points in time, diluted 10-fold in assay buffer and measured directly by ALB408-423 (SEQ ID NO 1) sandwich ELISA (triplicates). The signal decrease was calculated in % relative to the 0 min-value of ALB408-423 (= 100%) in each fluid. A) Calculated ng/ml-values of the 10-fold dilution incubated in the ELISA are shown. Standard deviations were calculated from the three values available (n=1, 1 donor, 1 day). B) Calculated %-values of signal decrease over time relative to 0 min-value of ALB408-423 (SEQ ID NO 1) in each fluid (results shown are representative).
Figure 10b: Detection of endogenous ALB408-423 (SEQ ID NO 1) in acidified Plasma. Plasma (obtained with lithium heparin tubes) was prepared freshly and acidified to pH 4 with 1 M HCl, then distributed equally to two tubes and incubated at RT for 0 to 72 hrs. Aliquots were stored at -80°C or 500 µl were used directly for centrifugation by 50 kDa MWCO filters. Flow through and retentate were collected and analyzed by ALB408-423 (SEQ ID NO 1) sandwich ELISA. Shown are the concentrations of summarized flow through and retentate calculated from the 1,000- and 10,000-fold dilution (n=1, results shown are representative for 3 donors as well as plasma (obtained with potassium EDTA tubes) and serum preparations. MWCO, molecular weight cut-off.
Figure 10c: The effect of a protease inhibitor of ALB408-423 (SEQ ID NO 1) production in acidified plasma was investigated. Protease inhibitor cocktail for acid proteases (5%), 5% DMSO (solvent), or PBS (phosphate buffered saline) was added to acidified plasma and incubated at RT for 0 to 168 hrs, stored at -80°C or directly centrifuged, and analyzed by ALB408-423 (SQ ID NO 1) sandwich ELISA. Concentrations of flow through and retentate were calculated from the 1,000-fold dilution, and summarized. Plasma (obtained with lithium heparin tubes) was prepared freshly and acidified to pH 4 with 1M HCl, then distributed equally to three tubes. Protease inhibitor cocktail for acid proteases (5%, Sigma P8340), 5% DMSO (solvent), or PBS was added to 14 ml plasma, then incubated at RT for 0 to 168 hrs. Aliquots were stored at -80°C or 500 µl were used directly for centrifugation via 50 kDa MWCO filters. Flow through and retentate were collected and analyzed by ALB408-423 (SEQ ID NO 1) sandwich ELISA. Shown are the concentrations of summarized flow through and retentate calculated from the 1000-fold dilution (n=1). DMSO, dimethyl sulfoxides; MWCO, molecular weight cut-off; PBS, phosphate buffered saline.

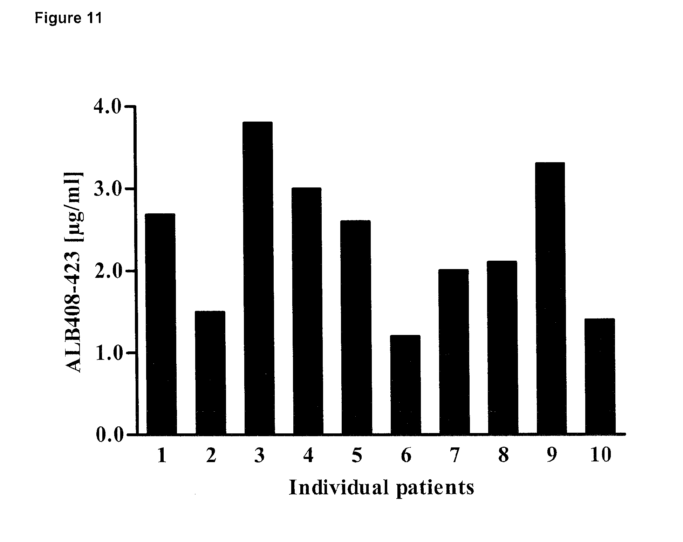

The quantification of ALB408-423 in different samples is shown in Figures 11 to 16.
Figure 11: ALB408-423 (SEQ ID NO 1) can be detected in unprocessed hemofiltrate. Hemofiltrate was diluted in 10-fold steps in assay buffer and measured by the ALB408-423 (SEQ ID NO 1) sandwich ELISA. ALB408-423-specific signals were detected in the µg/ml-range in different patients.

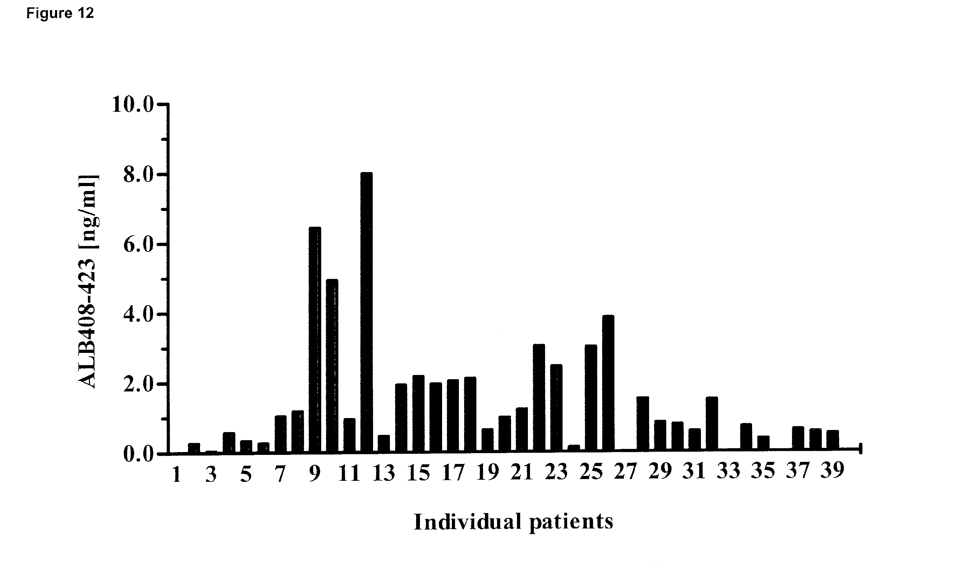

Figure 12: ALB408-423 (SEQ ID NO 1) can be detected in vaginal lavage. Vaginal lavage was diluted in 10-fold steps in assay buffer and measured by the ALB408-423 (SEQ ID NO 1) sandwich ELISA. ALB408-423-specific signals were detected in the ng/ml-range in different patients with varying signal strengths.

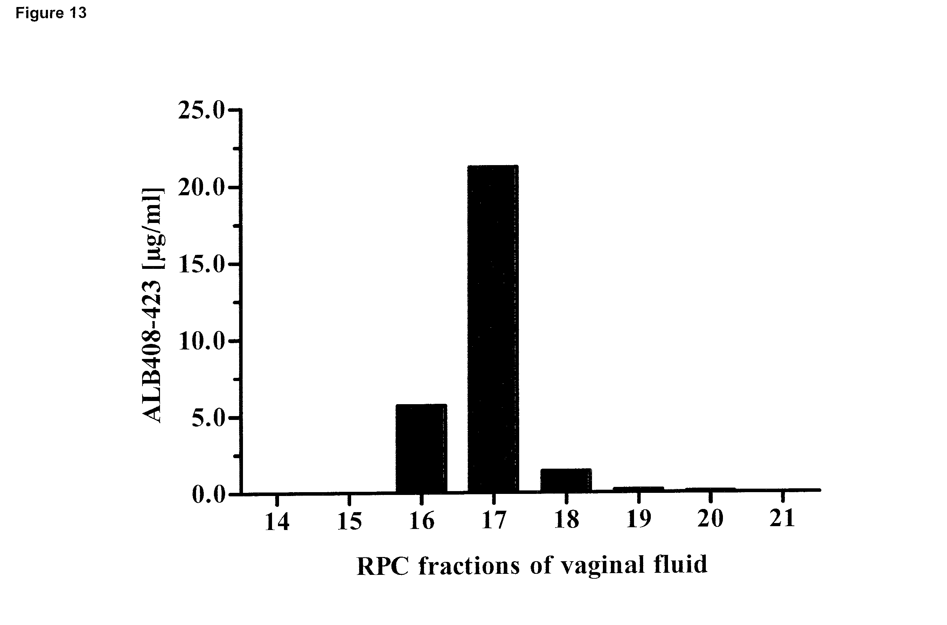

Figure 13: ALB-408-423 (SEQ ID NO 1) can be detected in fractions of reversed-phase chromatographed vaginal lavage. Lyophilized (i.e. reversed-phase chromatographed) vaginal lavage was reconstituted in ddH₂O, diluted in 10-fold steps and measured by the ALB408-423 (SEQ ID NO 1) sandwich ELISA. When calculating the total amounts of ALB408-423 in the unprocessed sample about 40 ng/ml ALB408-423 can be detected in vaginal secretion. (RPC, reversed-phase chromatography).

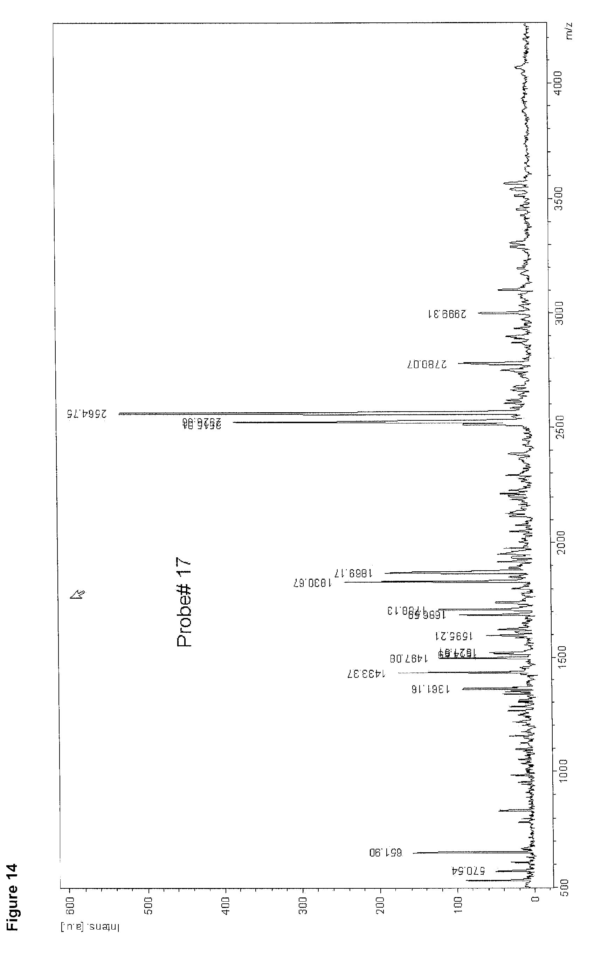

Figure 14: The presence of ALB408-423 (SEQ ID NO 1) in fractions of reversed-phase chromatographed vaginal lavage can be confirmed by mass spectrometry. About 76 ml vaginal lavage was reversed-phase chromatographed and lyophilized and reconstituted in ddH₂O. An ALB408-423-specific signal shows at 1830.67 Dalton detected in fraction 17.

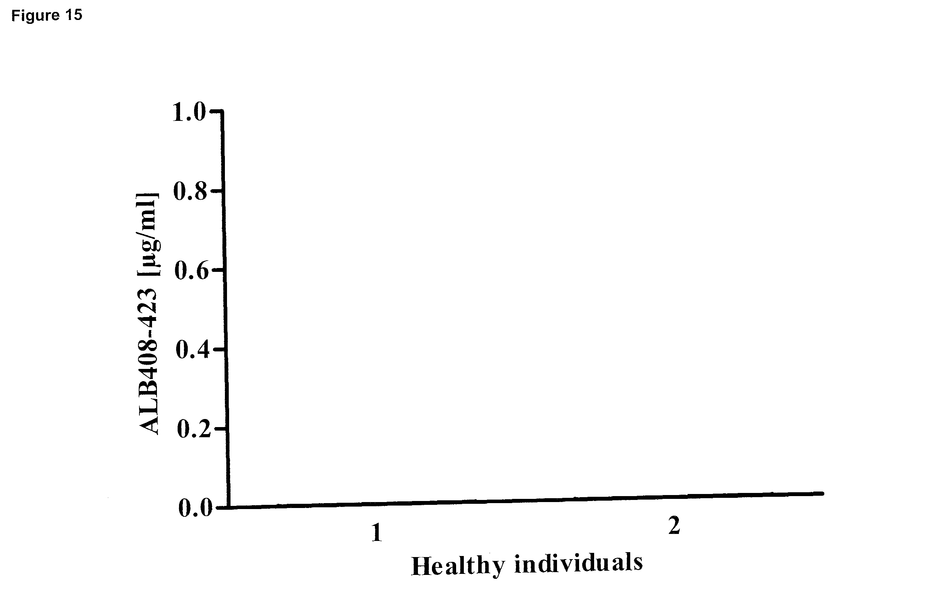

Figure 15: ALB408-423 (SEQ ID NO 1) can not be detected in urine samples from healthy individuals. Urine samples were diluted in 10-fold steps in assay buffer and measured by the ALB408-423 (SEQ ID NO 1) sandwich ELISA.

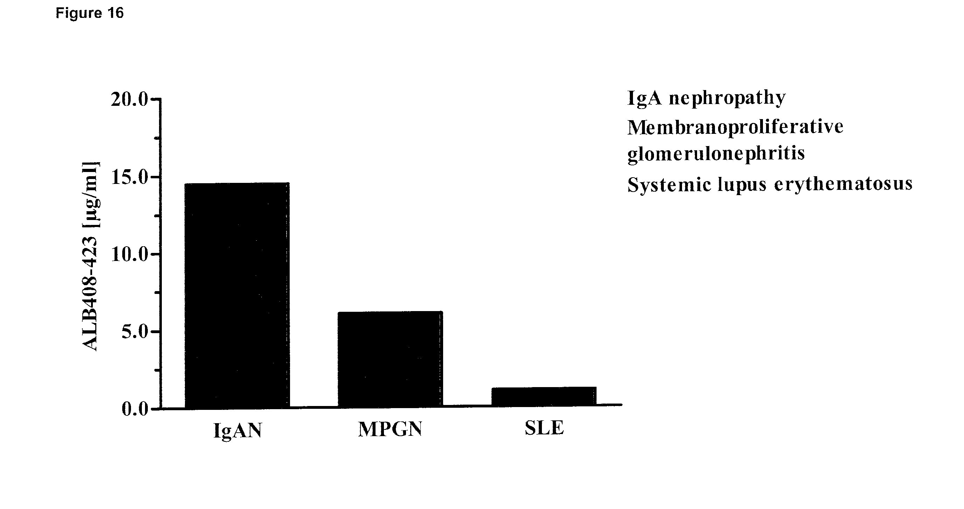

Figure 16: ALB408-423 (SEQ ID NO 1) can be detected in urine samples of patients with various kidney diseases. Urine samples were diluted in 10-fold steps in assay buffer and measured by the ALB408-423 (SEQ ID NO 1) sandwich ELISA, ALB408-423-specific signals were detected in the µg/ml-range in different patients with varying signal strengths. Urine samples from healthy individuals never showed a positive signal.

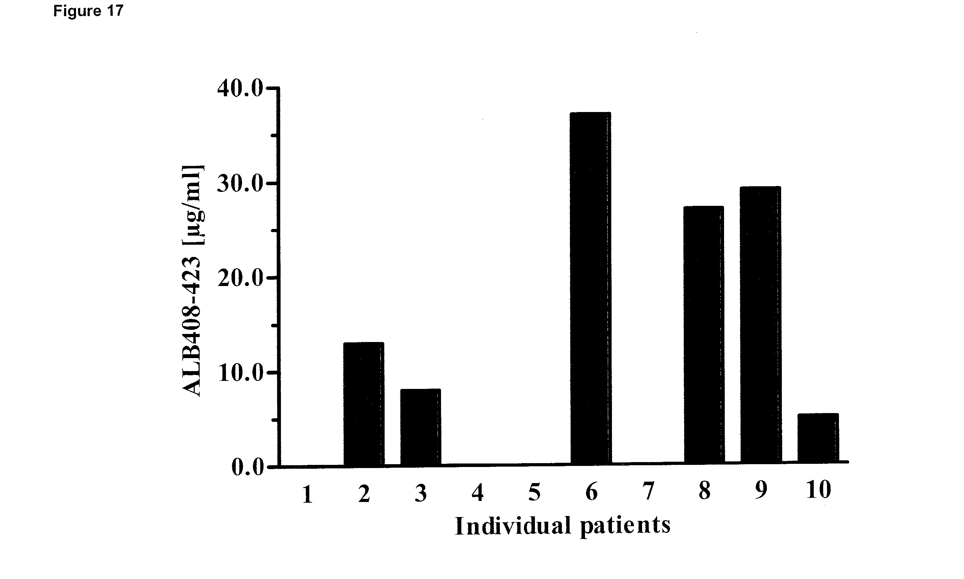

Figure 17: ALB408-423 (SEQ ID NO 1) can be detected in urine samples of patients with graft-versus-host disease. Urine samples were diluted in 10-fold steps in assay buffer and measured by the ALB408-423 (SEQ ID NO 1) sandwich ELISA. ALB408-423-specific signals were detected in the µg/ml-range in different patients with varying signal strengths.

The following non-limiting examples further illustrate the invention. It should be understood, however, that the invention as claimed should not be unduly limited to such specific embodiments. Indeed, various modifications of the described modes for carrying out the invention which are obvious to those skilled in the art are intended to be within the scope of the claims.

### Examples

In the examples patient samples (blood, urine, and vaginal lavage were obtained after approval of the study protocol by the local ethics committee. Blood and plasma samples were collected and further processed as usual.

### Example 1

### Generation of antibodies for the ALB408-423 (SEQ ID NO 1) sandwich ELISA

For the generation of specific antibodies against ALB408-423 (SEQ ID NO 1), rabbits and guinea pigs were immunized with the N-terminal half of ALB408-423 (LVRYTKKC, ALB408-415 (SEQ ID NO 2)), synthesized by Immunoglobe GmbH, Himmelstadt, Germany) and the C-terminal half (CPQVSTPTL, ALB416-423 (SEQ ID NO 3)), respectively. The antisera were depleted two times against ALB407-424 and ALB409-422 to achieve higher specificity for ALB408-423 (SEQ ID NO 1). The N-terminal rabbit antibody (IG1185) was used as the capture reagent whereas the C-terminal guinea pig antibody was biotinylated (GP-2603#1) and served as the first detecting reagent.

### Example 2

### ALB408-423 sandwich ELISA protocol

This protocol was used in order to obtain the figures described above.

Corning 96 well microplates were coated with capture reagent (100 µl/well, 100-250 ng/ml) in coating buffer, incubated 5 min shaking at 450 rpm and for 22 ± 4 h stationary at 4°C. The next day, 250 µl 1X blocking buffer was added for 1 ± 0.5 h stationary at room temperature (RT). The plate was washed 5 times with 1X washing buffer. To prevent leftover unspecific antibodies from binding, and to enhance specificity, freshly prepared matrix solution was added (50 µl/well) without letting the plate dry out.

Next, synthetic ALB408-423 (SEQ ID NO 1), as standard, and the sample were pipetted onto the plate (50 µl/well). A 1:800 dilution of the first detecting reagent (Biotin GP-2603#1) in assay buffer was also prepared and added in this step (50 µl/well). Plates were incubated for 2 h shaking at 450 rpm and RT. The contents were aspirated and after three washing steps, 100 µl/well of second detecting reagent (Streptavidin-HRP, 200 ng/ml, 1X) in assay buffer was prepared, added, and incubated for 30 min shaking at 450 rpm and RT. After five washing steps, 100 µl of TMB (3,3'5,5'-Tetramethylbenzidine) warmed to RT was added. After 20 -30 min, the reaction was stopped with 1 M HCl and the optical densities (ODs) were recorded within 10 min at 450 nm and 650 nm as a reference value using the ELISA microplate reader. As reference values for every ALB408-423 (SEQ ID NO 1) sandwich ELISA (calibration/standard curve) or to test the assay functionality, 2-fold dilutions of ALB408-423 (SEQ ID NO 1) starting from 5 ng/ml were prepared from a highly concentrated peptide stock solution. The last well was used as a negative control i.e. background value and contained assay buffer without peptide.

## Claims

1. An *in vitro* process for the immunological diagnosis of a human subject with regard to diseases affecting the kidneys or a risk of acquiring diseases affecting the kidneys or graft-versus-host disease or a risk of acquiring graft-versus-host disease by determining immunologically the presence and/or concentration of the peptide ALB408-423 (SEQ ID NO 1) in a sample comprising the steps of:
(a) contacting the sample with a capture reagent which is capable of specifically binding to ALB408-423 (SEQ ID NO 1),
(b) determining the presence and/or amount of ALB408-423 (SEQ ID NO 1) bound to capture reagent formed in step (a).

2. Process according to claim 1, wherein the diseases affecting the kidneys are selected from the group comprising: any renal disease, nephropathy, IgA nephropathy, proteinuria, uremia, and membranous glomerulonephritis.

3. Process according to any of the preceding claims, wherein the sample is selected from urine, blood, plasma, serum, vaginal lavage, semen, saliva, sweat, breast milk, and/or cerebrospinal fluid.

4. Process according to any of the preceding claims, wherein the capture reagent is a monoclonal or polyclonal antibody, which specifically binds to an epitope present in ALB408-423 (SEQ ID NO 1).

5. Process according to any of the preceding claims, wherein the process is a direct or indirect immunoassay selected from the group comprising: competitive binding assay, non-competitive binding assay, a radioimmunoassay, an enzyme-linked immunosorbent assay (ELISA), a sandwich assay, a gel diffusion immunodiffusion assay, an agglutination assay, a fluorescent immunoassay, a chemiluminescence immunoassay, a protein A or protein G immunoassay, an immunoelectrophoresis assay, Western Blot, Dot Blot and flow cytometry.

6. Process according to claims 1 to 4, wherein the step (b) comprises:
(i) contacting the first intermediate complex obtained in step (a) with a first detecting reagent, which is capable of specifically binding to ALB408-423 (SEQ ID NO 1), resulting in a second intermediate complex whereby the first detecting reagent can produce a signal,
(ii) measuring the detectable signal produced in the second intermediate complex obtained in step (i), wherein the intensity of the detectable signal corresponds to the concentration of ALB408-423 (SEQ ID NO 1) in a sample.

7. Process according to claim 6, wherein the detectable signal is optical density, fluorescence, luminescence and/or radioactive signal.

8. Process according to any of the preceding claims, wherein the first detecting reagent is a monoclonal or polyclonal antibody, which specifically binds to an epitope present in ALB408-423 (SEQ ID NO 1).

9. Process according to any of the preceding claims, wherein the capture reagent and the first detecting reagent are different monoclonal antibodies, whereby capture reagent and first detecting reagent each independently and specifically bind to different epitopes present in ALB408-423 (SEQ ID NO 1).

10. Process according to any of the preceding claims, wherein shorter-length variants of the sequence (SEQ ID NO 1) are not immunologically detected in the process of the present invention.

11. Process according to claim 10, wherein shorter-length variants are derivatives of the peptide ALB408-423 (SEQ ID NO 1) which lack from either the C- and/or the N-terminus up to 7 amino acids.

12. Process according to any of the preceding claims, wherein the detection range of ALB408-423 (SEQ ID NO 1) is within 0.4 to 5 ng/ml based on total volume of a sample.

13. An immunoassay kit for detecting ALB408-423 (SEQ ID NO 1) in a sample in a process according to any of the preceding claims comprising:
- a capture reagent that specifically binds to an epitope of ALB408-423 (SEQ ID NO 1),

14. An immunoassay kit according to claim 13, wherein the capture reagent does not cross-react with other proteins or peptides present in a sample.
